(19) Europäisches Patentamt
European Patent Office
Office européen des brevets

(11) Publication number: 0 221 720 B1

# (12) EUROPEAN PATENT SPECIFICATION

(45) Date of publication of the patent specification: 27.06.90

(51) Int. Cl.[5]: C07D 211/90, C07D 401/04, C07D 405/04, C07D 413/04, C07D 417/04, C07D 409/04, A61K 31/445

(21) Application number: 86308158.4

(22) Date of filing: 21.10.86

## (54) Dihydropyridines.

(30) Priority: 25.10.85 GB 8526411

(43) Date of publication of application: 13.05.87 Bulletin 87/20

(45) Publication of the grant of the patent: 27.06.90 Bulletin 90/26

(84) Designated Contracting States: AT BE CH DE ES FR GB GR IT LI LU NL SE

(56) References cited:
EP-A- 031 801
EP-A- 0 132 375
EP-A- 0 161 917
EP-A- 0 206 747
DE-A- 2 658 183

PROGRESS IN PHARMACOLOGY, vol. 5/1, 1982, pages 25-52, Stuttgart; R. MANNHOLD et al.: "Qualitative and quantitative structure-activity relationships of specific Ca antagonists"

The file contains technical information submitted after the application was filed and not included in this specification

(73) Proprietor: Pfizer Limited, Ramsgate Road, Sandwich Kent CT13 9NJ(GB)

(72) Inventor: Cross, Peter Edward, 21, Cherry Avenue, Canterbury Kent(GB)
Inventor: Alker, David, 1, Clive Road, Margate Kent(GB)
Inventor: Campbell, Simon Fraser, Grey Friars Upper Street Kingsdown, Deal Kent(GB)

(74) Representative: Moore, James William, Dr., Pfizer Limited Ramsgate Road, Sandwich Kent CT13 9NJ(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

This invention relates to certain dihydropyridines, specifically to certain 1,4-dihydropyridines having a hydroxy substituent in a thioether side chain attached to the 2-position, and derivatives thereof, which have utility as anti-ischaemic and antihypertensive agents.

The compounds of the invention reduce the movement of calcium into the cell and they are thus able to delay or prevent the cardiac contracture which is believed to be caused by an accumulation of intracellular calcium under ischaemic conditions. Excessive calcium influx during ischaemia can have a number of additional adverse effects which would further compromise the ischaemic myocardium. These include less efficient use of oxygen for ATP production, activation of mitochondrial fatty acid oxidation and possibly, promotion of cell necrosis. Thus the compounds are useful in the treatment or prevention of a variety of cardiac conditions, such as angina pectoris, cardiac arrhythmias, heart attacks and cardiac hypertrophy. The compounds also have vasodilator activity since they can inhibit calcium influx in cells of vascular tissue and they are thus also useful as antihypertensive agents and for the treatment of coronary vasospasm.

DE-A-2 658 183 describes dihydropyridine derivatives having a substituent at the 2-position which may be an alkylthiomethyl group. EP-A-132 375 describes dihydropyridine derivatives having a $-CH_2-O-Y-X$ substituent at the 2 position, Y being a $C_1$-$C_4$ hydro carbon group and X a nitrogen-containing heterocyclic ring. EP-A-031 801 describes dihydropyridine derivatives having a substituent at the 2-position which may be 2-(2-methoxyethoxy)methyl group.

According to the specification of our co-pending European patent application publication no. 161 917 there are described and claimed a number of dihydropyridine anti-ischaemic and anti hypertensive agents wherein the 2-position of the dihydropyridine ring is substituted with hydroxyalkoxymethyl groups. We have now discovered a further related series of dihydropyridine compounds having valuable therapeutic properties wherein the substituent in the 2 position is a hydroxy alkylthiomethyl group.

According to the invention, there are provided novel 1,4-dihydropyridine derivatives of the formula:-

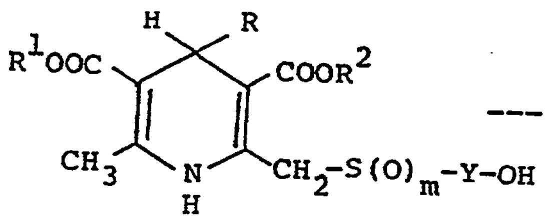

--- (I)

where

R is unsubstituted phenyl or phenyl substituted by one or two substituents each independently selected from nitro, halo, $C_1$-$C_4$ alkyl, $C_1$-$C_4$ alkoxy, hydroxy, trifluoromethyl and cyano;

$R^1$ and $R^2$ are each independently $C_1$-$C_4$ alkyl

Y is $-(CH_2)_n-$ where n is 2 or 3, $-CH_2CH(CH_3)-$ or $-CH_2C(CH_3)_2-$; and

m is 0, 1 or 2.

"Halo" means F, Cl, Br or I.

Alkyl and alkoxy groups having 3 or more carbon atoms can be straight or branched chain.

R is preferably phenyl substituted by 1 or 2 substituents selected from halo and $CF_3$, or is 2-chloropyrid-3-yl. R is most preferably 2-chlorophenyl or 2,3-dichlorophenyl.

$R^1$ and $R^2$ are preferably $CH_3$ or $C_2H_5$.

Most preferably, $R^1$ is $CH_3$ and $R^2$ is $C_2H_5$, or $R^1$ and $R^2$ are both $CH_3$.

Y is preferably $-(CH_2)_2-$, $-(CH_2)_4-$, $-CH_2CH(CH_3)-$ or $-CH_2C(CH_3)_2-$, most preferably $-(CH_2)_2-$.

Preferred compounds of the present invention are 4-(2,3-dichlorophenyl)-3-ethoxycarbonyl-2-[(2-hydroxyethyl)-thiomethyl]-5-methoxycarbonyl-6-methyl-1,4-dihydropyridine, and 3,5-bis (methoxycarbonyl)-4-(2-chlorophenyl)-2-[(2-hydroxyethyl)-thiomethyl]-6-methyl-1,4-dihydropyridine.

The compounds of the formula (I) containing one or more asymmetric centres will exist as one or more pairs of enantiomers, and such pairs or individual isomers may be separable by physical methods, e.g. by fractional crystallisation or chromatography of the parent compounds or suitable derivatives thereof as will be known to those skilled in the art. The invention includes the separated pairs as well as mixtures thereof, as racemic mixtures or as separated optically-active isomeric forms.

The compounds of the invention in which m is 0 can be prepared by reacting a compound of the formula:

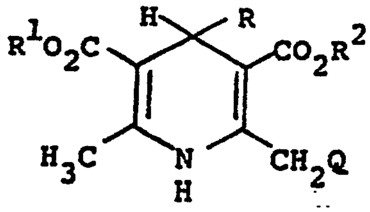 --- (II)

wherein R, $R^1$ and $R^2$ are as previously defined and Q is a leaving group, with a thiol of the formula:

HS-Y-OH --- (III)

wherein Y is as previously defined.

The leaving group Q may be for example a halo atom, preferably bromo, or a para-toluenesulphonyloxy, methanesulphonyloxy, trifluoromethanesulphonyloxy or l-imidazolylsulphonyloxy group. The reaction with the thiol of formula (III) is generally achieved by stirring the reactants in more or less equimolar proportions in an inert organic solvent e.g. tetrahydrofuran or N,N-dimethylformamide, in the presence of a base, e.g. potassium carbonate. The reaction is generally complete within a few hours at room temperature and the desired product is then isolated and purified using conventional techniques.

The compounds of the formula (I) in which m is 1 or 2, (i.e. the sulphinyl and sulphonyl derivatives) can be prepared by the oxidation of the corresponding thioether compounds, typically by using about one equivalent of a suitable oxidising agent such as metachloroperbenzoic acid or sodium metaperiodate, to give the sulphinyl compounds, or two or more equivalents to give the sulphonyl derivatives.

The starting materials of formula II are generally known compounds described in the literature or obtainable following literature precedents. Thus for example, in one process the corresponding 2-methyl-l,4-dihydropyridine may be halogenated to yield the corresponding compound of formula (II) wherein Q is halo. The reaction may for example be performed using pyridinium bromide perbromide to yield the 2-bromomethyl derivative.

In an alternative process the corresponding 2-hydroxymethyl-1,4-dihydropyridine may be converted to, for example, the 2-halomethyl, 2-paratoluenesulphonyloxymethyl, 2-methanesulphonyl or 2-trifluoromethyloxymethyl derivative of formula (II) by conventional methods. Thus, for example, in one particular variant of this process, reaction of 2-hydroxymethyl-1,4-dihydropyridine with sulphuryl chloride in N,N-dimethylformamide followed by addition of imidazole initially yields the compound of formula (II) wherein Q is a 1-imidazolylsulphonyloxy group. This compound in fact breaks down by reaction with the dihydropyridine nitrogen atom to yield a cyclic sulphamate ester which is reacted with the thiol of formula (III).

The ability of the compounds to inhibit the movement of calcium into the cell is shown by their effectiveness in reducing the contraction of vascular tissue in vitro which is the consequence of calcium influx caused by a high extracellular concentration of potassium ions. The test is performed by mounting spirally cut strips of rat aorta with one end fixed and the other attached to a force transducer. The tissue is immersed in a bath of physiological saline solution containing calcium ions at a concentration of 2.5 millimolar and potassium ions at a concentration of 5.9 millimolar. Potassium chloride solution is added to the bath with a pipette to give a final potassium ion concentration of 45 millimolar. The change in tension caused by the resulting contraction of the tissue is noted. The bath is drained and refilled with fresh saline solution containing the particular compound under test. After 45 minutes, the procedure is repeated and the contraction again noted. The concentration of compound required to reduce the response by 50% is determined.

The antihypertensive activity of the compounds is evaluated after oral administration by measuring the fall in blood pressure in spontaneously hypertensive rats or renally hypertensive dogs.

For administration to man in the curative or prophylactic treatment of cardiac conditions and hypertension, oral dosages of the compounds will generally be in the range of from 2-100 mg daily for an average adult patient (70 kg). Thus for a typical adult patient, individual tablets or capsules contain from 1 to 10 mg of active compound, in a suitable pharmaceutically acceptable vehicle or carrier. Dosages for intravenous administration would typically be within the range 1 to 10 mg per single dose as required. In practice the physician will determine the actual dosage which will be most suitable for an individual patient and it will vary with the age, weight and response of the particular patient. The above dosages are exemplary of the average case but there can, of course, be individual instances where higher or lower dosage ranges are merited, and such are within the scope of this invention.

For human use, the compounds of the formula (I) can be administered alone, but will generally be administered in admixture with a pharmaceutical carrier selected with regard to the intended route of administration and standard pharmaceutical practice. For example, they may be administered orally in the form of tablets containing such excipients as starch or lactose, or in capsules or ovules either alone or in admixture with excipients, or in the form of elixirs or suspensions containing flavouring or colouring agents. They may be injected parenterally, for example, intravenously, intramuscularly or subcutaneously. For

parenteral administration, they are best used in the form of a sterile aqueous solution which may contain other substances, for example, enough salts or glucose to make the solution isotonic with blood.

Thus in a further aspect the invention provides a pharmaceutical composition comprising a compound of the formula (I) together with a pharmaceutically acceptable diluent or carrier.

The invention also includes a compound of the formula (I) for use in medicine, in particular in the treatment of ischaemic heart disease, angina or hypertension in a human being.

The preparation of the compounds of the invention is illustrated by the following Examples.

EXAMPLE 1

4-(2,3-Dichlorophenyl)-3-ethoxycarbonyl-2-[(2-hydroxyethyl)-thiomethyl]-5-methoxycarbonyl-6-methyl-1,4-dihydropyridine

Sulphuryl chloride (0.80 ml) was added dropwise over 10 minutes to stirred, ice-cooled N,N-dimethylformamide (15 ml) and the mixture then added over 40 minutes to a stirred, ice-cooled solution of 4-(2,3-dichlorophenyl)-3-ethoxycarbonyl-2-hydroxymethyl-5-methoxycarbonyl-6-methyl-1,4-dihydropyridine (2.00 g) and imidazole (0.85 g) in N,N-dimethylformamide (30 ml). The mixture was stirred at room temperature for 2.5 hours and evaporated. The residue was dissolved in ethyl acetate and the solution washed twice with 2M hydrochloric acid, dried over magnesium sulphate and evaporated. The residue was dissolved in a mixture of tetrahydrofuran (15 ml) and N,N-dimethylformamide (15 ml) and the solution added to a stirred solution of 2-mercaptoethanol (0.95 ml) in a mixture of tetrahydrofuran (15 ml) and N,N-dimethylformamide (15 ml) containing potassium carbonate (2.00 g). The mixture was stirred at room temperature for 2 hours and evaporated. The residue was partitioned between ethyl acetate and water and the layers separated. The organic layer was washed twice with water and once with saturated brine, dried over magnesium sulphate and evaporated. The residue was purified by chromatography on silica (10 g) using hexane plus 50-100% dichloromethane as eluant. Appropriate fractions were combined and evaporated and the residue crystallised from di-isopropyl ether to give the title compound (0.60 g), m.p. 125°C. Found: C,52.11; H,4.98; N,3.01. $C_{20}H_{23}Cl_2NO_5S$ requires: C,52.17; H,5.03; N,3.04%.

EXAMPLE 2

3,5-Bis(methoxycarbonyl)-4-(2-chlorophenyl)-2-[(2-hydroxyethyl)-thiomethyl]-6-methyl-1,4-dihydropyridine

Pyridinium bromide perbromide (2.00 g) was added in one portion to a stirred, ice-cooled solution of 3,5-bis(methoxy-carbonyl)-4-(2-chlorophenyl)-2,6-dimethyl-1,4-dihydropyridine (1.68 g) and pyridine (0.80 ml) in dichloromethane (40 ml) and the mixture stirred at 0°C for 1.5 hours. The mixture was washed twice with ice-cold 0.5 M hydrochloric acid, dried over magnesium sulphate and evaporated without external heating. The residue was dissolved in tetrahydrofuran (15 ml) and the solution added to a stirred, ice-cooled solution of 2-mercaptoethanol (0.78 g) in tetrahydrofuran (20 ml) containing potassium carbonate (1.38 g). The mixture was stirred at 0°C for 2 hours and then at room temperature for 16 hours and then partitioned between ethyl acetate and water. The layers were separated and the organic layer washed with water, dried over sodium sulphate and evaporated. The residue was purified by chromatography on silica (17 g) using dichloromethane plus 0-50% ethyl acetate as eluant. Appropriate fractions were combined and evaporated to give the title compound (0.38 g) as an oil. Found: C,55.59; H,5.42; N,3.49. $C_{19}H_{22}ClNO_5S$ requires C,55.41; H,5.35; N,3.40%.

**Claims for the Contracting States: BE, CH, DE, FR, GB, IT, LI, LU, NL, SE**

1. 1,4-dihydropyridine derivatives of the formula (I):

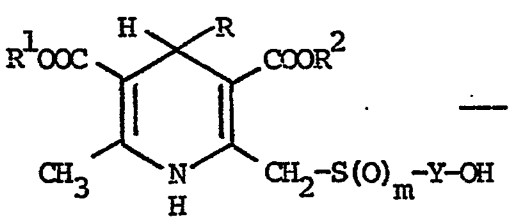

--- (I)

where

R is unsubstituted phenyl or phenyl substituted by one or two substituents each independently selected from nitro, halo, $C_1$-$C_4$ alkyl, $C_1$-$C_4$ alkoxy, hydroxy, trifluoromethyl and cyano;

$R^1$ and $R^2$ are each independently $C_1$-$C_4$ alkyl;

Y is $-(CH_2)_n-$ where n is 2 or 3, $-CH_2CH(CH_3)-$ or $-CH_2C(CH_3)_2-$; and

m is 0, 1 or 2.

2. A compound of formula (I) as claimed in claim 1 wherein R is 2-chlorophenyl or 2,3-dichlorophenyl.
3. A compound as claimed in any preceding claim wherein either $R^1$ is $CH_3$ and $R^2$ is $C_2H_5$ or $R^1$ is $C_2H_5$ and $R^2$ is $CH_3$.
4. A compound as claimed in any preceding claim wherein Y is $-(CH_2)_2-$ and m is 0.
5. 4-(2,3-dichlorophenyl)-3-ethoxycarbonyl-2-[(2-hydroxyethyl)thiomethyl]-5-methoxycarbonyl-6-methyl-1,4-dihydropyridine.
6. 3,5-Bis (methoxycarbonyl)-4-(2-chlorophenyl)-2-[(2-hydroxyethyl)-thiomethyl]-6-methyl-1,4-dihydropyridine.
7. A pharmaceutical composition comprising a compound of formula (I) as claimed in any preceding claim, together with a pharmaceutically acceptable diluent or carrier.
8. A compound of the formula (I) as defined in any one of claims 1 to 6, for use in medicine.
9. Use of a compound of the formula (I) as defined in any one of claims 1 to 6 in the manufacture of a medicament for the treatment of hypertension or ischaemia.

**Claims for the Contracting States: AT, ES, GR**

1. A process for preparing 1,4-dihydropyridine derivatives of the formula (I):

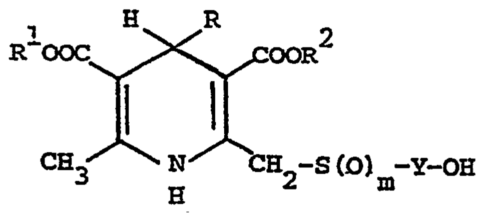

---(I)

where
R is unsubstituted phenyl or phenyl substituted by one or two substituents each independently selected from nitro, halo, $C_1$-$C_4$ alkyl, $C_1$-$C_4$ alkoxy, hydroxy, trifluoromethyl and cyano;
$R^1$ and $R^2$ are each independently $C_1$-$C_4$ alkyl;
Y is $-(CH_2)_n-$ where n is 2 or 3, $-CH_2CH(CH_3)-$ or $-CH_2C(CH_3)_2-$; and
m is 0, 1 or 2,
characterised by reaction of a compound of the formula (II):

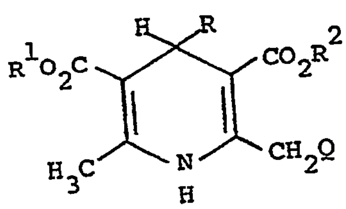

---(II)

wherein R, $R^1$ and $R^2$ are as previously defined and Q is a leaving group, with a thiol of formula (III):

HS-Y-OH ---(III)

wherein Y is as previously defined to give a compound of formula (I) in which m is 0 followed by optionally oxidising the compound of formula (I) in which m is 0 to give a compound wherein m is 1 or 2.
2. A process as claimed in claim 1 wherein Q is a halo, para-toluenesulphonyloxy, methanesulphonyloxy, trifluoromethanesulphonyloxy or 1-imidazolylsulphonyloxy group.
3. A process as claimed in any preceeding claim wherein R is 2-chlorophenyl or 2,3-dichlorophenyl.
4. A process as claimed in any preceeding claim wherein $R^1$ is $CH_3$ and $R^2$ is $C_2H_5$ or $R^1$ is $C_2H_5$ and $R^2$ is $CH_3$.
5. A process as claimed in any prceeding claim wherein Y is $-(CH_2)_2-$ and m is 0.
6. A process as claimed in any preceeding claim wherein the reaction is performed in the presence of a base.
7. A process as claimed in claim 6 wherein the base is potassium carbonate.
8. A process as claimed in claim 1 wherein the oxidising agent is present in an amount of one equivalent to give the sulphinyl compound or in an amount of two or more equivalents to give the sulphonyl derivatives.
9. A process as claimed in claim 1 wherein the oxidising agent is metachloroperbenzoic acid or sodium metaperiodate.

10. A process for preparing a pharmaceutical composition comprising mixing a compound of the formula (I) as defined in claim 1 with a pharmaceutically acceptable diluent or carrier.

**Patentansprüche für die Vertragsstaaten: BE, CH, DE, FR, GB, IT, LI, LU, NL, SE**

1. 1,4-Dihydropyridinderivate der Formel (I)

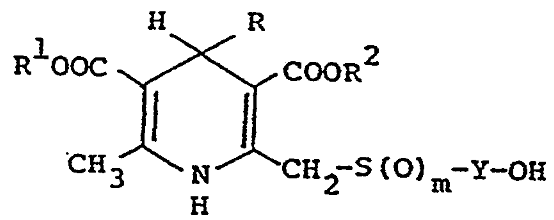

(I),

worin R unsubstituiertes Phenyl oder Phenyl substituiert mit einem oder zwei Substituenten jeweils unabhängig ausgewählt aus Nitro, Halogen, $C_1$-$C_4$-Alkyl, $C_1$-$C_4$-Alkoxy, Hydroxy, Trifluormethyl und Cyano bedeutet; $R^1$ und $R^2$ jeweils unabhängig $C_1$-$C_4$-Alkyl darstellen; Y die Bedeutung $-(CH_2)_n$, wobei n 2 oder 3 ist, $-CH_2CH(CH_3)-$ oder $-CH_2C(CH_3)_2-$ hat und m Null, 1 oder 2 ist.

2. Verbindung der Formel (I), wie in Anspruch 1 beansprucht, worin R 2-Chlorphenyl oder 2,3-Dichlorphenyl ist.

3. Verbindung, wie in einem vorhergehenden Anspruch beansprucht, worin $R^1$ $CH_3$ und $R^2$ $C_2H_5$ bedeuten oder $R^1$ $C_2H_5$ und $R^2$ $CH_3$ sind.

4. Verbindung, wie in einem vorhergehenden Anspruch beansprucht, worin Y $-(CH_2)_2-$ bedeutet und m Null ist.

5. 4-(2,3-Dichlorphenyl)-3-äthoxycarbonyl-2-[(2-hydroxyäthyl)-thiomethyl]-5-methoxycarbonyl-6-methyl-1,4-dihydropyridin.

6. 3,5-Bis-(methoxycarbonyl)-4-(2-chlorphenyl)-2-[(2-hydroxyäthyl)-thiomethyl]-6-methyl-1,4-dihydropyridin.

7. Pharmazeutische Zusammensetzung umfassend eine Verbindung der Formel (I), wie in einem vorhergehenden Anspruch beansprucht, zusammen mit einem pharmazeutisch annehmbaren Verdünnungsmittel oder Träger.

8. Verbindung der Formel (I), wie in einem der Ansprüche 1 bis 6 beansprucht, zur Verwendung in der Medizin.

9. Verwendung einer Verbindung der Formel (I), wie in einem der Ansprüche 1 bis 6 beansprucht, bei der Herstellung eines Medikaments zur Behandlung von Bluthochdruck oder Ischämie.

**Patentansprüche für die Vertragsstaaten: AT, ES, GR**

1. Verfahren zum Herstellen von 1,4-Dihydropyridinderivaten der Formel (I)

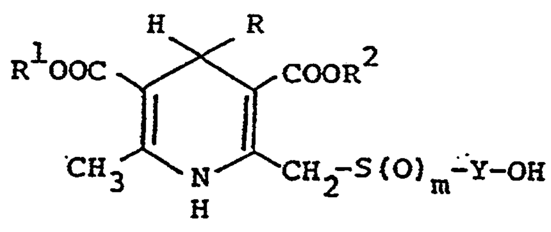

(I),

worin R unsubstituiertes Phenyl oder Phenyl substituiert mit einem oder zwei Substituenten jeweils unabhängig ausgewählt aus Nitro, Halogen, $C_1$-$C_4$-Alkyl, $C_1$-$C_4$-Alkoxy, Hydroxy, Trifluormethyl und Cyano bedeutet; $R^1$ und $R^2$ jeweils unabhängig $C_1$-$C_4$-Alkyl darstellen; Y die Bedeutung $-(CH_2)_n$, wobei n 2 oder 3 ist, $-CH_2CH(CH_3)-$ oder $-CH_2C(CH_3)_2-$ hat und m Null, 1 oder 2 ist, gekennzeichnet durch Umsetzen einer Verbindung der Formel (II)

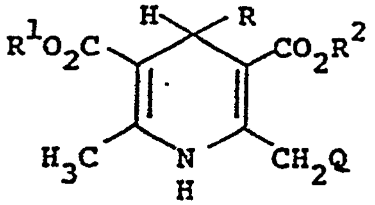

(II),

worin R, $R^1$ und $R^2$ wie oben definiert sind und Q eine abspaltbare Gruppe bedeutet, mit einem Thiol der Formel (III)

HS - Y - OH (III),

worin Y wie oben definiert ist, wobei eine Verbindung der Formel (I) erhalten wird, in der m Null ist, gegebenenfalls gefolgt vom Oxidieren der Verbindung der Formel (I), worin m Null ist, wobei eine Verbindung erhalten wird, in der m 1 oder 2 ist.

2. Verfahren, wie in Anspruch 1 beansprucht, worin Q Halogen, eine p-Toluolsulfonyloxy-, Methansulfonyloxy-, Trifluormethansulfonyloxy- oder 1-Imidazolylsulfonyloxygruppe ist.

3. Verfahren, wie in einem vorhergehenden Anspruch beansprucht, worin R 2-Chlorphenyl oder 2,3-Dichlorphenyl ist.

4. Verfahren, wie in einem vorhergehenden Anspruch beansprucht, worin $R^1$ $CH_3$ und $R^2$ $C_2H_5$ bedeuten oder $R^1$ $C_2H_5$ und $R^2$ $CH_3$ sind.

5. Verfahren, wie in einem vorhergehenden Anspruch beansprucht, worin Y $-(CH_2)_2-$ bedeutet und m Null ist.

6. Verfahren, wie in einem vorhergehenden Anspruch beansprucht, worin die Reaktion in Anwesenheit einer Base durchgeführt wird.

7. Verfahren, wie in Anspruch 6 beansprucht, worin die Base Kaliumcarbonat ist.

8. Verfahren, wie in Anspruch 1 beansprucht, worin das Oxidationsmittel in einer Menge von einem Äquivalent vorhanden ist, wobei die Sulfinylverbindung erhalten wird, oder in einer Menge von zwei oder mehreren Äquivalenten, wobei die Sulfonylderivate erhalten werden.

9. Verfahren, wie in Anspruch 1 beansprucht, worin das Oxidationsmittel m-Chlorperbenzoesäure oder Natriummetaperjodat ist.

10. Verfahren zum Herstellen einer pharmazeutischen Zusammensetzung, welches das Mischen einer Verbindung der Formel (I), wie in Anspruch 1 definiert, mit einem pharmazeutisch annehmbaren Verdünnungsmittel oder Träger umfaßt.

**Revendications pour les Etats contractants: BE, CH, DE, FR, GB, IT, LI, LU, NL, SE**

1 - Dérivés 1,4-dihydropyridine de formule (I):

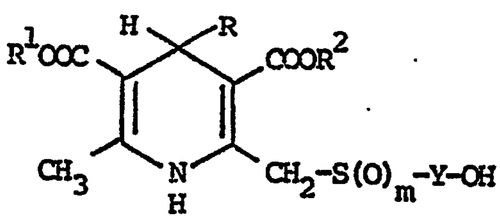

---(I)

dans laquelle
R est un radical phényle non substitué ou phényle substitué par un ou deux substituants identiques ou différents choisis parmi les radicaux nitro, halogéno, alkyle en $C_1$-$C_4$, alcoxy en $C_1$-$C_4$, hydroxy, trifluorométhyle et cyano ;
$R^1$ et $R^2$, identiques ou différents, représentent un radical alkyle en $C_1$-$C_4$ ;
Y représente $-(CH_2)_n-$ où n est égal à 2 ou 3, $-CH_2CH(CH_3)-$ ou $-CH_2C(CH_3)_2-$ ; et
m représente 0, 1 ou 2.

2 - Composé de formule (I) selon la revendication 1, dans laquelle R est un groupe 2-chlorophényle ou 2,3-dichlorophényle.

3 - Composé selon l'une des revendications précédentes dans laquelle soit $R^1$ représente $CH_3$ et $R^2$ représente $C_2H_5$ soit $R^1$ représente $C_2H_5$ et $R^2$ représente $CH_3$.

4 - Composé selon l'une quelconque des revendications précédentes, dans laquelle Y représente $-(CH_2)_2-$ et m représente 0.

5 - 4-(2,3-dichlorophényl)-3-éthoxycarbonyl-2-((2-hydroxyéthyl)thiométhyl)-3-méthoxycarbonyl-6-méthyl-1,4-dihydropyridine.

6 - 3,5-Bis (méthoxycarbonyl)-4-(2-chlorophényl)-2-((2-Hydroxyéthyl)-thiométhyl)-6-méthyl-1,4-dihydropyridine.

7 - Composition pharmaceutique comprenant un composé de formule (I) selon l'une quelconque des revendications précédentes, avec un diluant ou un véhicule pharmaceutiquement acceptable.

8 - Composé de formule (I) tel que défini selon l'une quelconque des revendications 1 à 6, pour son utilisation en médecine.

9 - Utilisation d'un composé de formule (I) selon l'une quelconque des revendications 1 à 6, dans la fabrication d'un médicament pour le traitement de l'hypertension ou de l'ischémie.

**Revendications pour les Etats contractants: AT, ES, GR**

1 - Procédé de préparation de dérivés 1,4-dihydropyridine de formule (I):

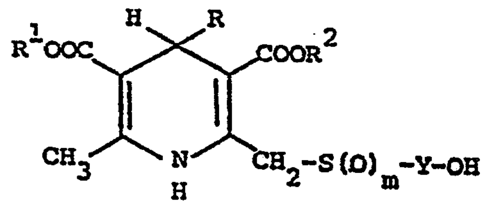

---(I)

dans laquelle
R est un radical phényle non substitué ou phényle substitué par un ou deux substituants identiques ou différents choisis parmi les radicaux nitro, halogéno, alkyle en $C_1$-$C_4$, alcoxy en $C_1$-$C_4$, hydroxy, trifluorométhyle et cyano ;
$R^1$ et $R^2$, identiques ou différents, représentent un radical alkyle en $C_1$-$C_4$ ;
Y représente $-(CH_2)_n-$ où n est égal à 2 ou 3, $-CH_2CH(CH_3)-$ ou $-CH_2C(CH_3)_2-$ ; et
m représente 0, 1 ou 2,
caractérisé par la réaction d'un composé de formule (II)

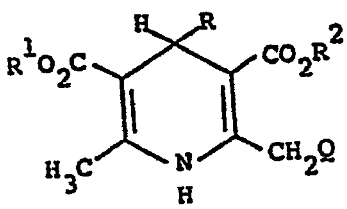

---(II)

dans laquelle R, $R^1$ et $R^2$ sont tels que précédemment définis et Q est un groupe labile, avec un thiol de formule (III)

HS-Y-OH (III)

dans laquelle Y est tel que défini précédemment pour donner un composé de formule (I) dans lequel m est égal à O, suivi de l'oxydation éventuelle du composé de formule (I) dans laquelle m représente 0 pour donner un composé dans lequel m représente 1 ou 2.

2 - Procédé selon la revendication 1, dans lequel Q est un groupe halogéno, para-toluènesulfonyloxy, méthanesulfonyloxy, trifluorométhanesulfonyloxy ou 1-imidazolylsulfonyloxy.

3 - Procédé selon l'une quelconque des revendications précédentes, dans lequel R est un groupe 2-chlorophényle ou 2,3-dichlorophényle.

4 - Procédé selon l'une quelconque des revendications précédentes, dans lequel $R^1$ représente $CH_3$ et $R^2$ représente $C_2H_5$ ou $R^1$ représente $C_2H_5$ et $R^2$ représente $CH_3$.

5 - Procédé selon l'une quelconque des revendications précédentes, dans lequel Y représente $-(CH_2)_2-$ et m représente 0.

6 - Procédé selon l'une quelconque des revendications précédentes, dans lequel la réaction est mise en œuvre en présence d'une base.

7 - Procédé selon la revendication 6, dans lequel la base est le carbonate de potassium.

8 - Procédé selon la revendication 1, dans lequel l'agent oxydant est présent à raison d'un équivalent pour donner le composé sulfinyle ou à raison de deux équivalents ou plus pour donner les dérivés sulfonyle.

9 - Procédé selon la revendication 1, dans lequel l'agent oxydant est l'acide métachloroperbenzoïque ou le métaperiodate de sodium.

10 - Procédé de préparation d'une composition pharmaceutique qui consiste à mélanger un composé de formule (I) tel que défini dans la revendication 1 avec un diluant ou un véhicule pharmaceutiquement acceptable.